# EUROPÄISCHE PATENTSCHRIFT

(11) **EP 4 009 947 B1**
(45) Veröffentlichungstag und Bekanntmachung des Hinweises auf die Patenterteilung: **26.06.2024**
(21) Anmeldenummer: 20781504.4
(22) Anmeldetag: 28.09.2020
(51) Int. Cl.: A61K 9/00, A61K 9/70, A61K 47/08, A61K 47/06, A61K 45/06, A61K 9/12, A61K 31/196, A61K 31/381, A61K 31/46, A61K 33/00

(54) **LAGTIME-VERKÜRZUNG/EISSPRAY**
LAG TIME REDUCTION/ICE SPRAY
RÉDUCTION DE TEMPS DE DÉCALAGE/PULVÉRISATION DE GLACE

(30) Priorität: 31.10.2019 DE 102019129444
(43) Veröffentlichungstag der Anmeldung: 15.06.2022
(73) Patentinhaber: LTS Lohmann Therapie-Systeme AG, 56626 Andernach (DE)
(72) Erfinder: RONNANDER, James Paul, Mount Vernon, New York 10550 (US); KOCH, Andreas, 56581 Melsbach (DE)
(74) Vertreter: Meissner Bolte Partnerschaft mbB
(86) Internationale Anmeldenummer: PCT/EP2020/077108
(87) Internationale Veröffentlichungsnummer: WO 2021/083593

(56) Entgegenhaltungen:
- DE-A1- 10 000 085
- DE-A1- 10 230 558
- DE-A1-102011 112 374
- US-A1- 2016 213 905
- Unknown: "Himani Fast Relief Herbal Pain Relief Spray 150ml + Ointment 2 x 50g", , 2018, XP055759169, Internet Gefunden im Internet: URL:https://www.luluhypermarket.com/en-ae/ himani-fast-relief-herbal-pain-relief-spra y-150ml-/p/1461378 [gefunden am 2020-12-11]
- M BHARKATIYA ET AL: "Skin penetration enhancement techniques", JOURNAL OF YOUNG PHARMACISTS, Bd. 1, Nr. 2, 1. Januar 2009 (2009-01-01), Seite 110, XP055759083, India ISSN: 0975-1483, DOI: 10.4103/0975-1483.55741

## Beschreibung

Die vorliegende Erfindung betrifft einen Kit, umfassend mindestens ein Kältespray und mindestens eine Darreichungsform zur transdermalen Verabreichung von mindestens einem pharmazeutisch aktiven Wirkstoff, wobei die mindestens eine Darreichungsform ein transdermales therapeutisches System umfasst, sowie die Verwendung dieses Kits in einem Verfahren zur Behandlung eines Patienten, wobei zunächst die Haut des Patienten an mindestens einer Stelle abgekühlt wird und anschließend eine Darreichungsform zur transdermalen Verabreichung von mindestens einem pharmazeutisch aktiven Wirkstoff auf die abgekühlte Stelle der Haut appliziert wird.

In einem Onlineshop der URL: https://www.luluhypermarket.com/en-ae/himani-fast-relief-herbal-painrelief-spray-150ml-/p/1461378 wurde am 2020-12-11 mit dem Himani Fast Relief Herbal Pain Relief Spray ein Spray zur schmerzlindernden Anwendung gefunden.

DE102011112374A1 beschreibt ein Verfahren zur wenigstens vorübergehenden, Straffung der menschlichen Haut, wobei auf die betroffenen Hautstellen kurzfristig ein Kältereiz aufgebracht wird, der die Temperatur dieser Hautstellen vorübergehend absenkt und so eine Straffung der menschlichen Haut bewirkt.

DE10000085A1 beschreibt ein Verfahren zur Verbesserung der Wirkung der EMLA

Creme, dadurch gekennzeichnet, dass nach Auftragung der EMLA Creme auf das Hautareal und Abdeckung mit der Tegaderm-Folie auf das Areal Kühlkompressoren aufgelegt oder Kaltluft oder ein Kältespray appliziert wird.

US2016/213905A1 beschreibt Geräte und Verfahren, die so konfiguriert sind, dass sie der Haut Wärme und Kälte zusammen mit einer Hautbehandlung zuführen, um die Geschwindigkeit des Hautbehandlungsflusses zur Haut zu steuern.

DE10230558A1 beschreibt eine Kombinationstherapie, die eine Dauerverabreichung eines Transdermalen Therapeutischen Systems (TTS) zur Basisversorgung eines Patienten mit einem pharmazeutischen Wirkstoff und eine anfängliche Verabreichung desselben pharmazeutischen Wirkstoffs mittels eines Sprays umfasst.

M. Bharkatiya et al.: "Skin penetration enhancement techniques", JOURNAL OF YOUNG PHARMACISTS, Bd. 1, Nr. 2, 1. Januar 2009 (2009-01-01), Seite 110, beschreibt Darreichungsformen zur transdermalen Verabreichung, die eine kontrollierte Abgabe eines Arzneimittels in den Körperkreislauf durch Permeation durch die Haut ermöglichen.

Darreichungsformen zur transdermalen Verabreichung von mindestens einem pharmazeutisch aktiven Wirkstoff haben in den letzten Jahren zur Behandlung zahlreicher Erkrankungen weite Verbreitung gefunden, da sie gegenüber anderen Verabreichungsformen mit Vorteilen verbunden sind.

So werden zum einen Magen, Darm und Leber durch Umgehung des Gastrointestinaltraktes geschont. Zum anderen kann der First-Pass-Effekt umgangen und die Compliance erhöht werden, da der Patient nicht regelmäßig Tabletten einnehmen muss. Ferner besteht die Möglichkeit einer kontinuierlichen und kontrollierten Freisetzung des Wirkstoffes über einen längeren Zeitraum hinweg ohne die Gefahr der Über- oder Unterdosierung gegenüber oralen Darreichungsformen wie Tabletten.

Darreichungsformen zur transdermalen Verabreichung von mindestens einem pharmazeutisch aktiven Wirkstoff haben jedoch den Nachteil, dass je nach Wirkstoff eine sogenannte Resorptionsverzögerungszeit bis zum Eintritt einer systemischen Wirkung (lag time) auftritt.

Diese kommt dadurch zustande, dass der transdermal applizierte Wirkstoff zuerst an den Ort seiner Resorption gelangen bzw. diffundieren muss; in diesem Fall die Wegstrecke von der Grenzschicht Darreichungsform-Stratum Corneum als oberste bzw. äußerste Hautschicht zum Stratum Papillare, in der die ersten Blutgefäße (oberflächliches Blutgefäßnetz) als Resorptionsorgan liegen.

Die Resorptionsverzögerungszeit (lag time) ist also diejenige Zeit, ab der ein gleichmäßiger Diffusionsstrom des Wirkstoffes aus der Darreichungsform und damit der Beginn der therapeutischen Wirkung eintritt. Je nach Diffusionsgeschwindigkeit der betreffenden Wirkstoffe kann diese lag time von 6 Stunden, wie beispielswiese bei Nicotin, bis zu 24 Stunden, wie bei Buprenorphin betragen.

Beim Buprenorphin hat die relativ lange Resorptionsverzögerungszeit (lag time) zum Beispiel die Konsequenz, dass ein Schmerzpatient, der von der oralen Buprenorphin-Tablette auf eine transdermale Applikation von Buprenorphin umgestellt werden soll, gleichzeitig mit der transdermalen Applikation unbedingt eine Bolus-Injektion bekommen muss, damit er bis zum Eintritt der systemischen Wirkung durch die transdermale Applikation schmerztherapeutisch gut versorgt ist. Aber auch für andere Indikationen ist ein möglichst schneller Wirkungseintritt erwünscht, wie beispielsweise die Behandlung von Schwindelanfällen oder Migräneattacken.

Verfahren zur Resorptionsverzögerungszeit bei der Applikation von Darreichungsformen zur transdermalen Verabreichung eines pharmazeutisch aktiven Wirkstoffs sind aus dem Stand der Technik bekannt.

So offenbart die WO WO2005/100603 A2 eine Vorbehandlung der Haut durch Tape-Stripping (Entfernung der obersten Zelllagenschichten des Stratum Corneum) und die WO 96/34566 A1 eine Vorbehandlung der Haut durch einen Laser.

Diese bekannten Verfahren zur Vorbehandlung der Haut haben aber den Nachteil, dass dabei die Haut irreversibel geschädigt wird.

Aufgabe der vorliegenden Erfindung war es die Resorptionsverzögerungszeit (lag time) bei der Applikation von Darreichungsformen zur transdermalen Verabreichung mindestens eines pharmazeutisch aktiven Wirkstoffs zu verkürzen, damit sich dadurch ein schnellerer konstanter Wirkstofffluss (=schnellerer Wirkungseintritt) einstellen kann, ohne dass dabei die Haut nachhaltig geschädigt wird.

Diese Aufgabe wurde überraschender Weise durch die Verwendung eines Kältesprays zur Verkürzung der Resorptionsverzögerungszeit (lagtime) bei der Applikation einer Darreichungsform zur transdermalen Verabreichung von mindestens einem pharmazeutisch aktiven Wirkstoff gelöst.

Es wurde gefunden, dass die Hautbarriere durch Behandlung mit einem Kältespray herabgesetzt werden und somit die Resorptionsverzögerungszeit (lag time) bei der nachfolgenden Applikation einer Darreichungsform zur transdermalen Verabreichung von mindestens einem pharmazeutisch aktiven Wirkstoff verkürzt werde kann.

Die vorliegende Erfindung betrifft daher einen Kit, umfassend mindestens ein Kältespray und mindestens eine Darreichungsform zur transdermalen Verabreichung von mindestens einem pharmazeutisch aktiven Wirkstoff, wobei die mindestens eine Darreichungsform ein transdermales therapeutisches System umfasst.

Im Folgenden kann "umfassen" auch "bestehend aus" bedeuten.

Im Rahmen der vorliegenden Erfindung werden die Begriffe "Resorptionsverzögerungszeit" und "lag time" synonym benutzt.

Im Rahmen der vorliegenden Erfindung werden auch die Begriffe "Kältespray" und "Eisspray" synonym benutzt.

Unter Kältespray versteht man ein in einer Spraydose abgefülltes Gas, das dazu dient Gegenstände oder Körperteile abzukühlen. Mit solchen bekannten Kältesprays werden vorzugsweise Temperaturen von -25 bis -55 °C erreicht. Vorzugsweise sind in den Kältesprays verschiedene Gase und Gasgemische vorhanden, wobei als Unterscheidungskriterien hauptsächlich die tiefste erreichbare Temperatur und die Brennbarkeit in Frage kommen.

Die mindestens eine Darreichungsform zur transdermalen Verabreichung von mindestens einem pharmazeutisch aktiven Wirkstoff umfasst erfindungsgemäß ein transdermales therapeutisches System.

Weitere bekannte Darreichungsformen, welche nicht beansprucht werden, umfassen ein Gel, eine Lotion, eine Salbe, eine Creme und/oder ein Mikronadelsystem.

Unter einem transdermalen therapeutischen System (auch transdermalem Pflaster genannt) versteht man ein auf die Haut aufzubringendes System mit festgelegter Applikationsfläche, das einen pharmazeutisch aktiven Wirkstoff, vorzugsweise nach Zeit und Menge, kontrolliert an den Körper eines Patienten abgeben kann.

Solche Systeme weisen in der Regel eine Deckfolie (backing layer) auf, welche das Pflaster und seinen Inhalt nach außen schützt und gegebenenfalls mit Informationen bedruckt ist. Zur Hautseite hin ist es vorzugsweise mit einer Abziehfolie versehen (release liner), die die klebrige Seite des Systems abdeckt. Die Abziehfolie wird vor dem Aufkleben des Systems entfernt und ist zwecks leichterem Ablösen oftmals silikonisiert.

Hinsichtlich der Technik der kontrollierten Wirkstoffabgabe aus dem System kam man zwischen Matrix-Systemen (Matrix-Pflastern) und Membran-Systemen (auch Reservoir-oder Depot-Systeme bzw. Reservoir- oder Depot-Pflaster genannt) unterscheiden.

Bei den Matrix-Systemen ist der Wirkstoff in einer aus einer oder mehreren Schichten bestehenden Matrix enthalten, die mit Hilfe einer Klebeschicht direkt auf der Haut aufliegt. Es sind auch Ausführungsformen möglich, bei denen die Matrix gleichzeitig die Klebeschicht ist. Die Diffusionsgeschwindigkeit des Wirkstoffes aus der Matrix heraus bestimmt die Resorptionsgeschwindigkeit. In einigen Ausführungsformen kann es zwischen Matrix- und Klebeschicht eine zusätzliche Membran geben, welche den Wirkstofffluss steuert.

Bei den Membran-Systemen liegt ein Reservoir des Wirkstoffs unter einer Trägerfolie, der aus dem Reservoir kontrolliert durch eine poröse Membran in die Haut abgegeben wird.

Die Vorteile eines transdermalen therapeutischen Systems sind auf Patientenseite eine sichere, zuverlässige, exakte und schmerzfreie Dosierung von pharmazeutisch aktiven Wirkstoffen und die einfachere Therapie von Kindern, älteren sowie pflegebedürftigen Patienten. Ferner sind transdermale therapeutische Systeme ideal für Patienten mit Schluckbeschwerden und bei verlängerten Einnahmeintervallen, insbesondere bei Mehrtagespflastern.

Die Vorteile eines transdermalen therapeutischen Systems sind auf Herstellerseite die mögliche Formulierung von pharmazeutisch aktiven Wirkstoffen mit nur geringer oraler Bioverfügbarkeit, eine kontrollierte, gleichmäßige Zufuhr von pharmazeutisch aktiven Wirkstoffen ohne Wirkstoffspitzen, eine gute Steuerungsmöglichkeit der Arzneimitteldosierung durch Variation der Fläche, kein Wirkstoffverlust durch Vermeidung des First-Pass-Metabolismus in der Leber und kein Abbau des Wirkstoffs im Magen-Darm-Trakt.

Gele umfassen in der Regel gelierte Flüssigkeiten. Sie werden vorzugsweise mit geeigneten Quellmitteln (Gelbildnern) hergestellt. Dazu gehören beispielsweise Cellulosen, Stärken, Carbomere, Gelatine, Xanthan, Bentonit, Agar und/oder Pektin.

Dabei wird zwischen hydrophilen und lipophilen Gelen unterschieden. Gele können durchsichtig oder undurchsichtig sein.

Weitere mögliche Bestandteile sind unter anderem Wasser, Propylenglykol, Antioxidantien, Lipide (bei Lipogelen), Aromastoffe, Süßungsmittel und/oder Konservierungsmittel.

Gele werden unter anderem für die lokale oder systemische Verabreichung von Wirkstoffen und für die feuchte Wundbehandlung verwendet.

Eine Lotion ist eine äußerlich anzuwendende flüssige wässrige oder wässrigalkoholische Zubereitung mit suspendierten oder emulgierten pharmazeutisch aktiven Wirkstoffen, sowie möglicherweise Hilfsstoffen.

Lotionen sind in der Regel flüssiger als Cremes bzw. Salben und lassen sich deshalb leichter großflächig auf die Haut auftragen.

Als Lotion wird eine äußerlich anzuwendende Öl-in-Wasser-Emulsion oder Wasser-in-Öl-Emulsion bezeichnet. Sie ist sehr leicht und schmiert nicht.

Lotionen werden unter anderem für die lokale oder systemische Verabreichung von Wirkstoffen und für die feuchte Wundbehandlung verwendet.

Eine Salbe, vorzugsweise eine Suspensionssalbe, ist eine halbfeste Zubereitung zur äußerlichen Anwendung. Salben bestehen vorzugsweise aus einer einphasigen Grundlage, in der feste oder flüssige Substanzen dispergiert sein können.

Es wird zwischen hydrophoben Salben, wasseraufnehmenden Salben und hydrophilen Salben unterschieden. Salben können auch Emulgatoren und Wasser enthalten.

Zur Herstellung von Salben können beispielsweise fette Öle, Fette, Wachse, Erdölprodukte wie Vaseline und Paraffine, Triglyceride und/oder Macrogole (PEG) verwendet werden.

Salben werden unter anderem für die lokale oder systemische Verabreichung von Wirkstoffen und für die feuchte Wundbehandlung verwendet.

Eine Creme ist eine halbfeste Zubereitung, in der Regel für die Anwendung auf der Haut.

Eine Creme ist vorzugsweise eine mehrphasige Zubereitung, die aus einer lipophilen und einer wässrigen Phase besteht und mindestens einen pharmazeutisch aktiven Wirkstoff enthält. Man unterscheidet zwischen einer hydrophilen Creme (Öl-in-Wasser) und einer lipophilen/hydrophoben Creme (Wasser-in-Öl).

Eine Creme wird unter anderem für die lokale oder systemische Verabreichung von Wirkstoffen und für die feuchte Wundbehandlung verwendet.

Ein Mikronadelsystem, auch Mikronadelarray genannt, ist ein System, umfassend eine Vielzahl an Mikronadeln an einem Träger. Die Nadeln, die vorzugsweise eine Länge von 100 µm bis 1000 µm aufweisen, können aus unterschiedlichen Materialien, wie Keramik, Stahl, Polymeren oder Silizium bestehen. Dabei kann der pharmazeutisch aktive Wirkstoff zum einen durch Mikroporen verabreicht werden, zum anderen können bioabbaubaren Polymere in den Nadeln eingesetzt werden und somit der pharmazeutisch aktive Wirkstoff durch das Auflösen der Nadeln verabreicht werden. Mikronadelsysteme sind beispielsweise in der WO 2019/115815 A1 oder der WO 2016/162449 A1 beschrieben.

Der mindestens eine pharmazeutisch aktive Wirkstoff ist prinzipiell nicht eingeschränkt. Bevorzugt umfasst der mindestens eine pharmazeutisch aktive Wirkstoff einen Wirkstoff für Indikationen, bei denen ein möglichst schneller Wirkungseintritt gewünscht ist.

Der erfindungsgemäße Kit ist vorzugsweise dadurch gekennzeichnet, dass der mindestens eine pharmazeutisch aktive Wirkstoff aus der Gruppe, bestehend aus Hypnotica, Sedativa, Antieleptica, Weckaminen, Psychoneurotropica, Neuro-Muskelblockern, Antispasmodica, Antihistaminica, Antiallergica, Cardiotonica, Antiarrhythmica, Diuretica, Hypotensiva, Vasopressoren, Antitussiva, Expectorantia, Analgetica, Thyroidhormonen, Sexualhormonen, Glucocorticoidhormonen, Antidiabetica, Antitumor-Wirkstoffen, Antibiotica, Chemotherapeutica, Narcotica, Antiparkinson-Wirkstoffen, Antialzheimer-Wirkstoffen und/oder Triptanen ausgewählt ist.

Besonders bevorzugt ist der pharmazeutisch aktive Wirkstoff aus der Gruppe, bestehend aus Antieleptica, Antihistaminica, Antiallergica, Analgetica oder Triptanen ausgewählt. Gerade bei diesen Indikationen spielt ein möglichst schneller Wirkungseintritt eine wichtige Rolle.

Der erfindungsgemäße Kit ist vorzugsweise dadurch gekennzeichnet, dass das Kältespray ein in einer Sprühdose abgefülltes Flüssiggas umfasst.

Vorzugsweise umfasst das Flüssiggas Propan, Butan, Pentan, Tetrafluorethan, Dimethylether und/oder Chlorethan.

Ein zweckmäßiges Kältespray ist beispielsweise Servisol^{®} Freeze IT 20 von Servisol, Bridgwater, UK, auf Basis einer Mischung von Tetrafluorethan und Dimethylether.

Die vorliegende Erfindung betrifft ferner den vorstehend beschriebenen Kit zur Verwendung in der Behandlung eines Patienten.

Die vorliegende Offenbarung betrifft auch die nicht erfindungsgemäße Verwendung eines Kältesprays zur Verkürzung der Resorptionsverzögerungszeit (lag time) bei der Applikation einer Darreichungsform zur transdermalen Verabreichung von mindestens einem pharmazeutisch aktiven Wirkstoff.

Unter Resorptionsverzögerungszeit (lag time) wird, wie vorstehend beschrieben, die Zeit, ab der ein gleichmäßiger Diffusionsstrom des Wirkstoffes aus der Darreichungsform zur transdermalen Verabreichung von mindestens einem pharmazeutisch aktiven Wirkstoff und damit der Beginn der therapeutischen Wirkung eintritt, verstanden.

Die vorstehend bezüglich des Kits dargelegten Definitionen und vorteilhaften Ausführungsformen mit den damit verbundenen Vorteilen gelten analog für die vorstehend genannte nicht erfindungsgemäße Verwendung eines Kältesprays.

Die nicht erfindungsgemäße Verwendung eines Kältesprays ist vorzugsweise dadurch gekennzeichnet, dass die Darreichungsform ein transdermales therapeutisches System, ein Gel, eine Lotion, eine Salbe und/oder ein Mikronadelsystem umfasst.

Die nicht erfindungsgemäße Verwendung eines Kältesprays ist vorzugsweise dadurch gekennzeichnet, dass der mindestens eine pharmazeutisch aktive Wirkstoff einen Wirkstoff für Indikationen, bei denen ein möglichst schneller Wirkungseintritt gewünscht ist.

Die nicht erfindungsgemäße Verwendung eines Kältesprays ist vorzugsweise dadurch gekennzeichnet, dass der mindestens eine pharmazeutisch aktive Wirkstoff aus der Gruppe, bestehend aus Hypnotica, Sedativa, Antieleptica, Weckaminen, Psycho-neurotropica, Neuro-Muskelblockern, Antispasmodica, Antihistaminica, Antialler-gica, Cardiotonica, Antiarrhythmica, Diuretica, Hypotensiva, Vasopressoren, Antitussiva, Expectorantia, Analgetica, Thyroidhormonen, Sexualhormonen, Gluco-corticoidhormonen, Antidiabetica, Antitumor-Wirkstoffen, Antibiotica, Chemo-therapeutica, Narcotica, Antiparkinson-Wirkstoffen, Antialzheimer-Wirkstoffen und/oder Triptanen ausgewählt ist.

Besonders bevorzugt ist der pharmazeutisch aktive Wirkstoff aus der Gruppe, bestehend aus Antieleptica, Antihistaminica, Antiallergica, Analgetica oder Triptanen ausgewählt.

Das Kältespray ist in einer bevorzugten Ausführungsform dadurch gekennzeichnet, dass das Kältespray ein in einer Sprühdose abgefülltes Flüssiggas umfasst.

Das Kältespray umfasst vorzugsweise Propan, Butan, Pentan, Tetrafluorethan, Dimethylether und/oder Chlorethan.

Die vorliegende Erfindung betrifft auch ein Kit, wie vorstehend definiert in einem Verfahren zur Behandlung eines Patienten, umfassend die Schritte:
- Abkühlen mindestens einer Stelle der Haut des Patienten durch Behandlung mit einem Kältespray und
- Auftragen einer Darreichungsform zur transdermalen Verabreichung von mindestens einem pharmazeutisch aktiven Wirkstoff auf die abgekühlte Stelle der Haut, wobei die mindestens eine Darreichungsform ein transdermales therapeutisches System umfasst.

Die Abkühlung bewirkt eine Herabsetzung der Hautbarriere und damit eine verkürzte Resorptionsverzögerungszeit. Die Abkühlung erfolgt reversibel. Reversibel bedeutet, dass insbesondere kein Gefrierbrand (schonende Abkühlung) und insbesondere keine makroskopische Hautschädigung erzeugt wird.

Das Kit zu Verwendung in diesem Verfahren ist vorzugsweise dadurch gekennzeichnet, dass die Haut des Patienten durch Behandlung mit einem Kältespray abgekühlt wird.

Dabei gelten die vorstehenden Definitionen und bevorzugten Ausführungsformen für das Kältespray auch analog für das erfindungsgemäße Kit zur Verwendung in diesem Verfahren.

Das erfindungsgemäße Kit zur Verwendung in diesem Verfahren ist vorzugsweise dadurch gekennzeichnet, dass die Behandlung mit einem Kältespray für 3 sec bis 20 sec, vorzugsweise für 5 sec bis 15 sec, besonders bevorzugt für höchstens 10 sec erfolgt. Dadurch wird sichergestellt, dass die Herabsetzung der Hautbarriere reversibel ist.

Das erfindungsgemäße Kit zur Verwendung in diesem Verfahren ist vorzugsweise dadurch gekennzeichnet, dass zwischen dem Abkühlen (Kältebehandlung) mindestens einer Stelle der Haut des Patienten und der Applikation einer Darreichungsform zur transdermalen Verabreichung von mindestens einem pharmazeutisch aktiven Wirkstoff auf die abgekühlte Stelle der Haut weniger als 15 sec, vorzugsweise weniger als 10 sec vergehen. Wird diese Zeit überschritten, so ist der vorteilhafte Effekt der Herabsetzung der Hautbarriere nicht mehr ausreichend gegeben.

Das erfindungsgemäße Kit zur Verwendung in diesem Verfahren ist vorzugsweise dadurch gekennzeichnet, dass der Abstand des Sprühkopfes des Kältesprays zu der Haut des Patienten mindestens 15 cm, vorzugsweise 15 cm bis 30 cm beträgt. Damit wird sichergestellt, dass die Haut des Patienten nicht irreversibel geschädigt wird.

Besonders bevorzugt erfolgt die Behandlung mit einem Kältespray für höchstens 10 s und einem Abstand des Sprühkopfes des Kältesprays zu der Haut des Patienten von mindestens 15 cm.

Das erfindungsgemäße Kit zu Verwendung in diesem Verfahren ist vorzugsweise dadurch gekennzeichnet, dass die Darreichungsform ein transdermales therapeutisches System, ein Gel, eine Lotion, eine Suspensionssalbe und/oder ein Mikronadelsystem umfasst.

Dabei gilt das vorstehend Beschriebene bezüglich der einzelnen Darreichungsformen analog für das erfindungsgemäße Kit zur Verwendung in diesem Verfahren.

Das erfindungsgemäße Kit zur Verwendung in diesem Verfahren ist vorzugsweise dadurch gekennzeichnet, dass durch das Abkühlen der Haut eines Patienten bevor eine Darreichungsform zur transdermalen Verabreichung von mindestens einem pharmazeutisch aktiven Wirkstoff auf die abgekühlte Stelle der Haut appliziert wird eine Verkürzung der Resorptionsverzögerungszeit um mindestens den Faktor 1,3, vorzugsweise um mindestens den Faktor 1,4, besonders bevorzugt um mindestens den Faktor 1,7 und ganz besonders bevorzugt um mindestens den Faktor 2,0 erreicht wird.

### Figuren:

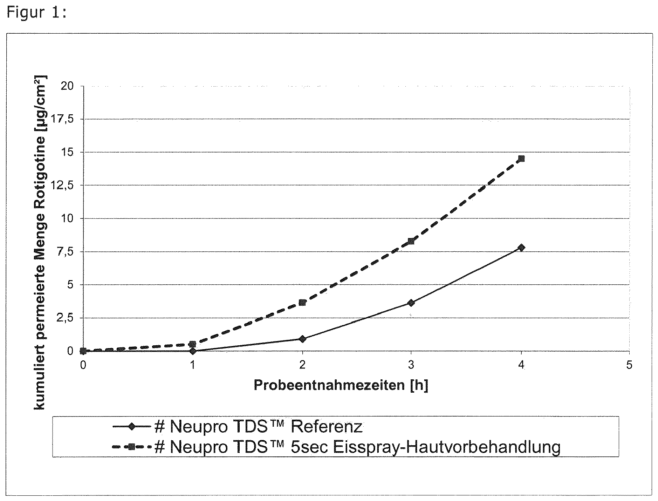

Figur 1 zeigt die Ergebnisse der Messung der *in vitro* Humanhautpermeation bei der Applikation von Probestanzlingen des transdermalen therapeutischen Systems Neupro^{®} (10 cm² TTS von der Firma UCB Pharma GmbH, Deutschland, mit einer Abgaberate von 2 mg/24h und einem Gehalt von 4,5 mg Wirkstoff, PZN 10393704) zur Verabreichung von Rotigotine mit und ohne einer vorhergehenden Hautbehandlung mit einem Kältespray. Die Resorptionsverzögerungszeit wurde um den Faktor 1,4 verringert.

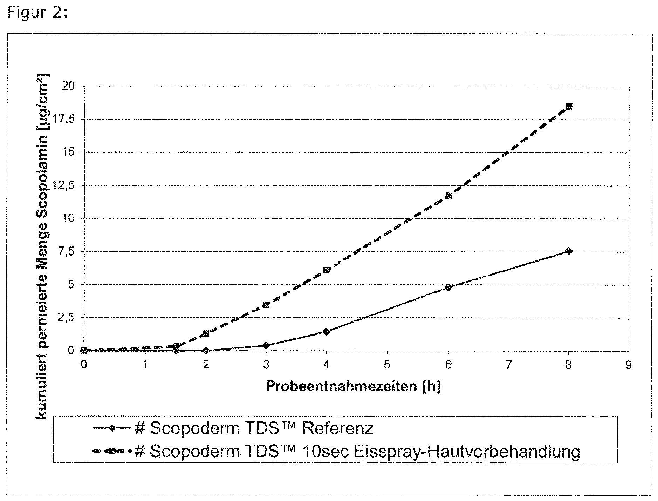

Figur 2 zeigt die Ergebnisse der Messung der *in vitro* Humanhautpermeation bei der Applikation von Probestanzlingen des transdermalen therapeutischen Systems Scopoderm^{®} (2,5 cm² TTS von der Firma Novartis Consumer Health GmbH, Deutschland, mit einer Abgaberate von 1,0 mg/72h und einem Gehalt von 1,54 mg Wirkstoff, PZN 00107146) zur Verabreichung von Scopolamin mit und ohne einer vorhergehenden Hautbehandlung mit einem Kältespray. Die Resorptionsverzögerungszeit wurde um den Faktor 2,0 verringert.

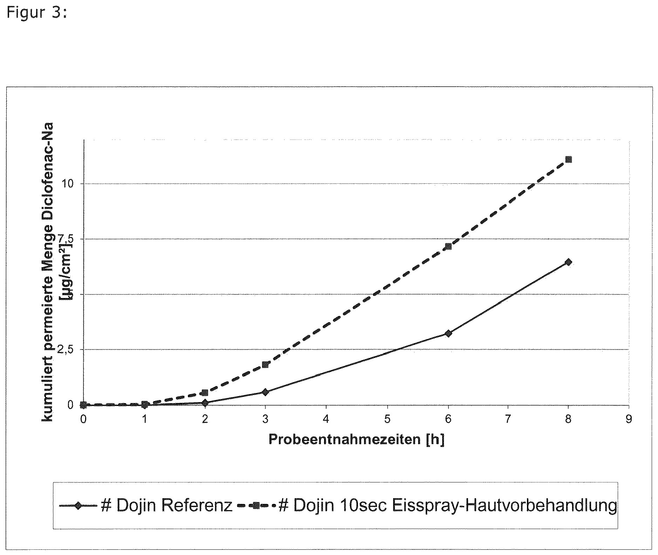

Figur 3 zeigt die Ergebnisse der Messung der *in vitro* Humanhautpermeation bei der Applikation des transdermalen therapeutischen Systems Voltaren Tape 30 Dojin^{®} (140 cm² Patch von Iyaku-Kako Co Ltd, Japan, mit einem Gehalt von 30 mg WIrkstoff; eine Abgaberate kann für dieses System nicht angegeben werden, da es sich um ein topisches System handelt. Da das Produkt nur im asiatischen Raum zugelassen und auch nur dort vertrieben wird, kann keine PZN angegeben werden). zur Verabreichung von Diclofenac-Na mit und ohne einer vorhergehenden Hautbehandlung mit einem Kältespray. Die Resorptionsverzögerungszeit wurde um den Faktor 2,0 verringert.

Die Erfindung wird nachfolgend anhand nicht beschränkender Beispiele näher erläutert.

### Beispiel 1:

Es wurde die Herabsetzung der Hautbarriere durch Messung des TEWL (transepidermaler Wasserverlust) bestimmt. Je höher dieser TEWL-Wert ist, umso stärker ist die Hautbarriere herabgesetzt.

Dazu wurden TEWL-Werte mittels einer Messsonde vom Typ AquaFlux200 der Fa. Biox, London, UK, bestimmt. Als Probe diente jeweils 800 µm dermatomisierte Human-Bauchhaut einer 71 Jahre alten Spenderin, die für eine bestimmte Zeit mit dem Kältespray Servisol^{®} Freeze IT 20 (Fa. Servisol Bridgewater, Somerset, UK) aus einer Entfernung von etwa 15 cm behandelt wurde. Die Messergebnisse vor und nach Kältespraybehandlung sind in Tabelle 1 zusammengefasst.

**Tabelle 1**

| Versuchs-Nr. | Behandlungsart | TEWL vor Kältebehandlung [g/m²xh] | TEWL nach Kältebehandlung [g/m²xh] | Steigerung TEWL [g/m²xh] | TEWL nach 30 min [g/m²xh] |
|---|---|---|---|---|---|
| 1 | 5 sec Kältespray | 15,63 | 46,90 | 31,28 | 14,71 |
| 2 | 5 sec Kältespray | 14,91 | 52,29 | 37,38 | 18,74 |
| 3 | 10 sec Kältespray | 13,54 | 56,84 | 43,30 | 16,42 |
| 4 | 10 sec Kältespray | 14,44 | 47,73 | 32,99 | 13,15 |

Eine intakte Hautbarriere ist laut der deutschen Gesellschaft für Dermatopharmazie durch einem TEWL-Bereich von 10 bis 24 g/m²xh charakterisiert. Die Werte in der sechsten Spalte zeigen, dass der Prozess reversibel ist und zu keiner Hautschädigung führt.

### Beispiel 2:

Die *in vitro* Humanhautpermeation von drei kommerziell erhältlichen transdermalen therapeutischen Systemen ohne und mit vorheriger Kältebehandlung wurde bestimmt. Dazu wurden die Hautstanzlinge komplett über ihre Oberfläche für 10 sec aus einer Entfernung von etwa 15 cm mit einem Kältespray (Servisol^{®} Freeze IT 20) behandelt. Nach etwa 10 sec (es bildet sich ein Eisfilm, der vorzugsweise erst wieder verschwunden sein sollte) wurde das entsprechende transdermale therapeutische System auf die behandelte Stelle der Haut aufgetragen und das Permeationsprofil (kumuliert permeierte Permeationsmenge in Abhängigkeit von der Zeit) mit Hilfe einer Franz-Zelle gemessen.

Im Donor-Kompartiment der Franz-Zelle befand sich das transdermale therapeutische System. Das Akzeptor-Kompartiment der Franz-Zelle wurde mit Puffer oder anderen Lösungen gefüllt. Durch regelmäßige Probennahme aus dem Akzeptor-Kompartiment konnte die Permeation einer Substanz über den gewählten Zeitraum durch die Haut hindurch verfolgt werden. Die Verwendung der Franz-Zelle als Diffusionsmodell ist vor allem geeignet, den Transport von Arzneistoffen durch humane Haut (= Permeation) vorherzusagen, was der systemischen Verfügbarkeit entspricht.

Hierbei wurden auf die epidermale Seite (Hautoberfläche) von 800 µm dermatomisierten kreisrunden Hautstücken mit einem Durchmesser von 25 mm jeweils 1,17 cm² große kreisrunde Stanzlinge der transdermalen therapeutischen Systeme aufgeklebt, einmal nach und einmal ohne (Referenz) Hautvorbehandlung. Als Akzeptormedium diente ein wässriger isotonischer Phosphatpuffer pH = 5,5 plus 0,1% Natriumazid als Konservierungsmittel mit einem Füllvolumen von 10 ml. Die Messung der Permeation wurde bei einer Temperatur von 32°C durchgeführt und die Werte nach 1, 2, 3 und 4 Stunden für Neupro^{®}, nach 1.5, 2, 3, 4, 6 und 8 Stunden für Scopoderm^{®} und nach 1, 2, 3, 6 und 8 Stunden für Dojin^{®} bestimmt. Die jeweiligen Messergebnisse mit den transdermalen therapeutischen Systemen Neupro^{®}, Scopoderm^{®} und Dojin^{®} sind den Figuren 1, 2 und 3 zu entnehmen.

## Patentansprüche

1. Kit, umfassend mindestens ein Kältespray und mindestens eine Darreichungsform zur transdermalen Verabreichung von mindestens einem pharmazeutisch aktiven Wirkstoff, wobei die mindestens eine Darreichungsform ein transdermales therapeutisches System umfasst.

2. Kit gemäß Anspruch 1, **dadurch gekennzeichnet, dass** der mindestens eine pharmazeutisch aktive Wirkstoff aus der Gruppe, bestehend aus Hypnotica, Sedativa, Antieleptica, Weckaminen, Psycho-neurotropica, Neuro-Muskelblockern, Antispasmodica, Antihistaminica, Antialler-gica, Cardiotonica, Antiarrhythmica, Diuretica, Hypotensiva, Vasopressoren, Antitussiva, Expectorantia, Analgetica, Thyroidhormonen, Sexualhormonen, Glucocorticoidhormonen, Antidiabetica, Antitumor-Wirkstoffen, Antibiotica, Chemo-therapeutica, Narcotica, Antiparkinson-Wirkstoffen, Antialzheimer-Wirkstoffen und/oder Triptanen ausgewählt ist.

3. Kit gemäß irgendeinem der vorhergehenden Ansprüche, **dadurch gekennzeichnet, dass** das Kältespray ein in einer Sprühdose abgefülltes Flüssiggas umfasst.

4. Kit gemäß Anspruch 3, **dadurch gekennzeichnet, dass** das Flüssiggas Propan, Butan, Pentan, Tetrafluorethan, Dimethylether und/oder Chlorethan umfasst.

5. Kit gemäß irgendeinem der vorhergehenden Ansprüche zur Verwendung in der Behandlung eines Patienten.

6. Kit gemäß einem der Ansprüche 1 bis 5 zur Verwendung in einem Verfahren zur Behandlung eines Patienten, umfassend die Schritte
- Abkühlen mindestens einer Stelle der Haut des Patienten durch Behandlung mit einem Kältespray und
- Auftragen einer Darreichungsform zur transdermalen Verabreichung von mindestens einem pharmazeutisch aktiven Wirkstoff auf die abgekühlte Stelle der Haut, wobei die mindestens eine Darreichungsform ein transdermales therapeutisches System umfasst.

7. Kit zur Verwendung gemäß Anspruch 6, **dadurch gekennzeichnet, dass** die Behandlung mit einem Kältespray für 3 sec bis 20 sec erfolgt.

## Claims

1. A kit, comprising at least one cold spray and at least one dosage form for transdermally administering at least one pharmaceutically active ingredient, wherein the at least one dosage form comprises a transdermal therapeutic system.

2. The kit according to claim 1, **characterised in that** the at least one pharmaceutically active ingredient is selected from the group consisting of hypnotics, sedatives, antiepileptics, analeptics, psychoneurotropic drugs, neuromuscular blockers, antispasmodics, antihistamines, antiallergics, cardiotonics, antiarrhythmics, diuretics, hypotensives, vasopressors, antitussives, expectorants, analgesics, thyroid hormones, sexual hormones, glucocorticoid hormones, antidiabetics, antitumour drugs, antibiotics, chemotherapeutics, narcotics, anti-Parkinson drugs, anti-Alzheimer drugs and/or triptans.

3. The kit according to any one of the preceding claims, **characterised in that** the cold spray comprises a liquid gas filled in a spray can.

4. The kit according to claim 3, **characterised in that** the liquid gas comprises propane, butane, pentane, tetrafluoroethane, dimethyl ether and/or chloroethane.

5. The kit according to any one of the preceding claims for use in the treatment of a patient.

6. Kit according to one of claims 1 to 5 for the use in a method for the treatment of a patient comprising the steps of
- cooling at least one area of the patient's skin by treatment with a cold spray and
- applying a dosage form for transdermally administering at least one pharmaceutically active ingredient to the cooled area of the skin, wherein the at least one dosage form comprises a transdermal therapeutic system.

7. Kit for the use according to claim 6, **characterised in that** the treatment with a cold spray lasts for between 3 sec and 20 sec.

## Revendications

1. Kit, comprenant au moins un aérosol de froid et au moins une forme pharmaceutique pour l'administration transdermique d'au moins un principe actif pharmaceutique, dans lequel l'au moins une forme pharmaceutique comprend un système thérapeutique transdermique.

2. Kit selon la revendication 1, **caractérisé en ce que** l'au moins un principe actif pharmaceutique est choisi dans le groupe constitué des hypnotiques, sédatifs, antiépileptiques, weckamines, psycho-neurotropes, bloqueurs neuromusculaires, antispasmodiques, antihistaminiques, antiallergiques, cardiotoniques, antiarythmiques, diurétiques, hypotensifs, vasopresseurs, antitussifs, expectorants, analgésiques, hormones thyroïdiennes, hormones sexuelles, hormones glucocorticoïdes, antidiabétiques, principes actifs antitumoraux, antibiotiques, agents chimiothérapeutiques, narcotiques, principes actifs antiparkinsoniens, principes actifs antialzheimer et/ou triptans.

3. Kit selon l'une quelconque des revendications précédentes, **caractérisé en ce que** l'aérosol de froid comprend un gaz liquéfié versé dans un flacon pulvérisateur.

4. Kit selon la revendication 3, **caractérisé en ce que** le gaz liquéfié comprend du propane, butane, pentane, tétrafluoroéthane, diméthyléther et/ou chloroéthane.

5. Kit selon l'une quelconque des revendications précédentes pour son utilisation dans le traitement d'un patient.

6. Kit selon l'une quelconque des revendications 1 à 5 pour son utilisation dans un procédé pour le traitement d'un patient, comprenant les étapes
- de refroidissement d'au moins un point de la peau du patient par traitement avec un aérosol de froid et
- d'application d'une forme pharmaceutique pour l'administration transdermique d'au moins un principe actif pharmaceutique sur le point refroidi de la peau, dans lequel l'au moins une forme pharmaceutique comprend un système thérapeutique transdermique.

7. Kit pour son utilisation selon la revendication 6, **caractérisé en ce que** le traitement s'effectue avec un aérosol de froid pendant 3 sec à 20 sec.
